# EUROPEAN PATENT SPECIFICATION

(11) **EP 3 071 140 B1**
(45) Date of publication and mention of the grant of the patent: **06.01.2021**
(21) Application number: 14863173.2
(22) Date of filing: 21.11.2014
(51) Int. Cl.: A61L 27/04, A61L 24/00

(54) **MACROPOROUS GRANULES OF ALKALINE EARTH PHOSPHATES OBTAINED WITH CEMENT TECHNOLOGY AND GAS EVOLVING POROGEN**
MAKROPORÖSE GRANULATE AUS ERDALKALIPHOSPHATEN DURCH ZEMENTTECHNOLOGIE UND GASERZEUGENDER PORENBILDNER HERGESTELLT
GRANULES MACROPOREUSES DE PHOSPHATES ALCALINO-TERREUX OBTENUES À L'AIDE D'UNE TECHNOLOGIE DE CIMENT ET D'UN AGENT POROGÈNE À ÉVOLUTION GAZEUSE

(30) Priority: 21.11.2013 US 201361907182 P
(43) Date of publication of application: 28.09.2016
(73) Proprietor: The University of Toledo, Toledo, OH 43606 (US)
(72) Inventor: BHADURI, Sarit, B., Toledo, OH 43606 (US); ZHOU, Huan, Toledo, OH 43606 (US); AGARWAL, Anand, K., Toledo, OH 43606 (US); GOEL, Vijay, K., Toledo, OH 43606 (US); SALEH, Sameh, Toledo, OH 43606 (US)
(74) Representative: Comoglio, Elena
(86) International application number: PCT/US2014/066761
(87) International publication number: WO 2015/077531

(56) References cited:
- WO-A1-2004/078223
- WO-A1-2009/110917
- WO-A1-2013/116457
- CN-A- 1 193 614
- US-A- 5 171 720
- US-A- 5 374 427
- US-A- 6 087 024
- US-A1- 2013 039 990
- US-B2- 6 953 594
- US-B2- 7 887 831
- BOHNER M ET AL: "Calcium Phosphate Emulsions", SIXTH WORLD BIO- MATERIALS CONGRESS TRANSACTIONS (15-20/5 2000 - TRANSACTIONS OF THE SIXTH WORLD BIOMATERIALS CONGRESS : MAY 15 - 20, 2000, HILTON WAIKOLOA VILLAGE RESORT, KAMUELA (BIG ISLAND), HAWAII, U.S.A, SOCIETY FOR BIOMATERIALS, US, vol. 1, 1 January 2000 (2000-01-01), page 20, XP008184854,
- LI: "Effects of ions in aqueous media...", JOURNAL OF APPLIED BIOMATERIALS, vol. 4, 1993, pages 221-229,

## Description

### BACKGROUND OF THE INVENTION

Cancellous bone, also known as trabecular bone, is a type of osseus tissue that makes up bones. Compared to the other type of osseus tissue, called cortical bone or compact bone, cancellous bone is less dense and therefore has a higher surface area to mass ratio. While cortical bone is a dense collection of lamellar sheets composed of mineralized collagen fibers and surrounded by vascular channels, cancellous bone is a highly porous material with high surface area and low density (typically about 0.2-0.5 g/cm³). Cancellous bone has about 75-85% porosity with pores having diameters of about 200-700 µm, while cortical bone has only 5-10% porosity with pores having diameters of about 1-100 µm. Given this difference, cancellous bone is more suitable for metabolic activity, such as the exchange of cations, and is highly vascular. Cancellous bone is found at the ends of long bones, proximal to joints, and in the interior of vertebrae. It would be advantageous for a bone implant, such as a hardened cement mass, to mimic the porosity and pore size of cancellous bone.

Porosity and pore size in bone implants are important. The pores of a bone implant, such as a hardened cement mass, should have a diameter of at least 50 µm, preferably at least 100 µm, in order to promote ingrowth of bone into the implant. Methods currently known in the art for creating porosity in bone implants, such as freeze-casting of ceramics, require long wait times for the sintering process. There is a need in the art for an injectable cement composition with macroporosity that can be produced in real-time, at the site and time of the desired application, rather than having to wait for a sintering process to create macroporosity. There is a need to develop these granules in a time-effective and cost-effective method.

The technology of producing granules has existed for several decades. Granules have been fabricated from various calcium phosphate phases such as hydroxylapatite (HA), tricalcium phosphate (TCP), calcium-deficient hydroxyapatite (CDHA), dibasic calcium phosphate dihydrate (DCPD), silicon-substituted apatite, and biphasic calcium phosphates. While some efforts have utilized coral-derived granules or bovine trabecular materials, conventional methods involve preparation of slurries of chemical origin of various phases of calcium phosphates, followed by granulation and final sintering. Due to immunological issues associated with allografts, it is preferred that artificial chemicals are used for the manufacturing of these granules. The drawbacks of the conventional processes include very little presence of pores, or too small of granules. To increase the pore content, polymers are sometimes added, which requires high temperature burn-off, followed by sintering. During the burn-off, the polymers melt and may not retain their original shape and size. The results of such methods produce granules of high porosity content, up to 75%, with pore sizes as large as 400 µm. Introduction of polymers has thus become important, but not necessarily yielding the best results.

It is apparent that several criteria need to be met for the successful development of porous granules. First, an effective porogen should be chosen, preferably in a particular form, that can be cleanly removed from the system, while leaving the shapes of pores intact, without collapsing the surrounding struts around them. Second, the granules should be of relevant size, such 1 mm - 2 mm in orthopedic applications and 0.25 mm - 1 mm in periodontal surgery applications. Third, the process should be simple, preferably involving a single step. Fourth, it is preferable that no high temperature sintering is needed in order to simplify the process, while simultaneously addressing contamination-related issues. Fifth, it is also preferable that there is enough radio-opacity present. Finally, the handling strength should be such that the granules do not crumble either during the processing or when exposed to body fluids.

Recent developments in this field include the use of sodium chloride (NaCl) as a porogen because NaCl is both leachable and biocompatible. In fact, the use of NaCl as a porogen is a well known concept in fabricating biopolymeric scaffolds, such as polycaprolactone, gelatin, polyurethane, and polylactic-co-glycolic acid (PLGA). NaCl has also been used as a porogen in the production of granules in orthopedic cement technology. The resulting granules have poorly crystallized apatite as the predominant phase, with the granule being in the size range of 2 mm - 4 mm, containing 50% porosity, and having pore sizes between 50 µm - 550 µm. While there have been recent attempts to improve the technique, no new progress has been made to create granules of the correct sizes containing macropores of desired dimensions and fabricated at room temperature without the use of high temperature sintering. Thus, there remains a need in the art for new macroporous cement compositions, and new methods of making macroporous cement compositions.

### SUMMARY OF THE INVENTION

The invention is defined by the claims. Provided herein is a macroporous cement composition for orthopedic or dental applications comprising pores made from a hydrogen-evolving process that occurs in a solution containing a mixture of a post-microwaved powder and a liquid, where the pores range in size from about 100 µm to about 1 mm, and wherein the macroporous cement composition comprises a magnesium phosphate that has been irradiated, the macroporous cement composition being injectable during the hydrogen-evolving process. The hydrogen-evolving process is a reaction between a metal and water present in the liquid, wherein the metal comprises magnesium. In particular embodiments, the magnesium is in the form of spherical particles. In particular embodiments, the magnesium particles have a diameter of about 45 µm. In particular embodiments, the magnesium is present in an alloy with one or more of Al, Zn, or a rare-earth metal. In certain embodiments, the liquid further comprises silica. In certain embodiments, the macroporous cement composition further includes a biopolymer or polysaccharide. In particular embodiments, the macroporous cement composition includes alginate. In particular embodiments, the macroporous cement composition includes chitosan.

The macroporous cement composition comprises a magnesium phosphate. In certain embodiments, the composition comprises a strontium-doped magnesium phosphate. In certain embodiments, the macroporous cement composition is injectable during the hydrogen-evolving process.

In certain embodiments, the macroporous cement composition further includes drug molecules in the pores. In certain embodiments, the macroporous cement composition further includes an antibiotic. In certain embodiments, the antibiotic is selected from the group consisting of clindamycin, tigecycline, vancomycin, ciprofloxacin, ofloxacin, sulfamethoxazole, trimethoprim/sulfamethoxazole, amoxicillin, penicillin V, penicillin G, procaine penicillin, benzathine penicillin, carbencillin, mezlocillin, ampicillin, piperacillin, bacampicillin, tiearcillin, ticarcillin, piperacillin/tazobactam, aztreonam, cefotetan, loracarbef, mefoxin, merrem, levofloxacin, lomefioxacin, primaxim, cycloserine, kanamycin, dicloxacillin, demeclocycline, minocycline, doxycycline, oxytetracycline, tobramycin, gentamicin, neomycin, amikacin, craramyein, nebcin, erythromycin/sulfisoxazole, netromycin, streptomycin, tobramycin, cefotaxime, cefuroxime, cefazoline, ceffibuten, ceffizoxime, cefaclor, cefopoerazone, cefprozil, cefadroxil monohydrate, ceftazidime, trimethoprim/sulfamethoxazole, cephalexin, cefazolin, cefamandole nafate, cefepime, cefonicid, sulfadiazine, norfloxacin, enoxacin, cefdinir, seromycin, ceftriaxone, cefixime, ceftazidime, clarithromycin, dirithromycin, methenamine, ethionamide, trovafioxacin, sparfloxacin, interfon-α, indinavir, ganciclovir, foscamet, lamivudine, famciclovir, rimantadine, zalcitabine, interferon-β, indinavir, ganciclovir, saquinavir, ritonavir, ribavirin, erythromycin, troleandomycin, azithromycin, eliiidamycin, colistin, amphotericin B, flucytosine, fluconazole, griseofulvin, grepafloxacin, ultramicrosize griseofulvin, terbinafine, ketoconazole, clotrimazole, dapsone, delavirdine, ziduvudine, amantadine, palivizumab, valacyclovir, didanosine, nelfinavir, nevirapine, ribavirin, cidofovir, pyrimethamine, metronidazole, furazolidone, atovaquone, stavudine, lamiduvine, acyclovir, mionazole, nystatin, itraconazole, chloroquine, pyrimethamine, mefloquine, hydroxychloroquine, capreomycin, permethrin, crotamiton, lindane, fluoro-uracil, ethambutol, rifabutin, isoniazid, aminosalicyclic acid, rifapentine, pyrazinamide, coenzoyl peroxide, chlorhexidine gluconate, sodium oxychlorosene, benzoyl peroxide, rifampin, rifampin/isoniazid, rifampin/isoniazid/pyrazinamide, nitrofurantoin, linezolid, nitrofurantoin, fosfomycin, nalidixic acid, atropine, oxytetracycline/sulfamethizole/phenazopyridine, mupirocin, chloramphenical, neomycin/polymyxin, tfimetorpim/polythyxin, tobramycin/dexamethasone, vidatabine, ciprofloxacin, ofioxacin, sulfacetamide, mupirocin, povidoneodine, gentamicin, nystatin, chloramphenicol, bacitracin, sulconazole, terbinafine, tetrachlorosalicylanilide, metronidazole, metromdazole, ciclopiroxolamine, clotrimazole, clotrimazole/betamethasone, butenafine, clotrimazole, nattifine, oxiconazole, selenium, econazole, penciclovir, and pharmaceutically acceptable salts thereof.

In certain embodiments, the macroporous cement composition further includes a growth factor. In particular embodiments, the growth factor is selected from the group consisting of BMP1, BMP2, BMP3, BMP4, BMP5, BMP6, BMP7, BMP8a, BMP8b, BMP10, or BMP15.

Further provided is a method for making a cement composition for orthopedic or dental applications, the method including the steps of mixing a powder component with a metal and a liquid component to form a gaseous solution, wherein the metal comprises magnesium and reacts with water in the liquid component to produce hydrogen gas bubbles in the gaseous solution; and allowing the gaseous solution to set into a hardened porous mass, wherein the hydrogen gas bubbles create pores in the hardened mass.

In certain embodiments of the methods described herein, at least one of alginate or chitosan is mixed with the powder component, the metal, and the liquid component In certain embodiments, the hardening of the gaseous solution occurs simultaneously with the production of hydrogen bubbles. In certain embodiments, the cement composition is extruded through a syrgine while pores are forming from the hydrogen bubbles.

Various aspects of this invention will become apparent to those skilled in the art from the following detailed description of the preferred embodiment.

### BRIEF DESCRIPTION OF THE DRAWING

**FIGS. 1A-1B****:** Photographs of a macroporous cement composition hardening in a test tube.
**FIGS. 2A-2F****:** Photographs of hardened cement granules with macro-sized pores visible.
**FIG. 3****:** X-ray diffraction pattern of a macroporous cement composition.
**FIGS. 4A-4J****:** SEM images of hardened macroporous cement compositions. Various sizes of pores are seen with the aid of the scale provided in each image.

### DETAILED DESCRIPTION OF THE INVENTION

The present disclosure relates to a one-step process for making macroporous cement compositions, such as those from alkaline earth phosphates, or granules with macroporosity for orthopedics and dental applications. Provided herein are compositions, and methods for making such compositions, involving the use of a gas-evolving process to create porosity in a granule. In accordance with the present disclosure, granules containing macropores are produced using a particular type of porogen in any suitable cement composition. This disclosure is particularly useful with, though by no means limited to, the bone cement compositions disclosed in PCT Publication No. WO 2013/116457 A1 dated August 8, 2013, which is PCT application PCT/US13/24040 filed on January 31, 2013, entitled "Injectable, Biodegradable Bone Cements And Methods Of Making and Using Same." However, the referenced PCT publication does not describe a process for making granules with macropores. The ability to produce granules with macropores is important in both orthopedics and in dentistry. Granules having pore sizes similar to those in human bones can act as three-dimensional scaffolds for cell delivery in tissue engineering, and are also effective in drug and growth factor delivery.

This disclosure represents improvements for producing granules containing macropores using various cement technology and compositions. Though calcium phosphate or magnesium phosphate cements, such as those disclosed in WO 2013/116457 A1, are often mentioned for illustrative purposes, it is to be understood that a gas-evolving process can be used to create macroporosity in any cement composition that involves the use of a liquid. Therefore, suitable final cement compositions include, but are not limited to, Ca-P cements such as hydroxyapatite [Ca₁₀(PO₄)₆(OH)₂], tetracalcium phosphate (TTCP, Ca₄(PO₄)₂O), tricalcium phosphate [a-TCP, α-Ca₂(PO₄)₂ and β-TCP, β-Ca₃(PO₄)₂], dicalcium phosphate anhydrous (DCPA, monetite, CaHPO₄), di-calcium phosphate dehydrate (DCPD, brushite, CaHPO₄.2H₂O), octacalcium phosphate (OCP, Ca₈H₂(PO₄)₆·5H₂O); Mg-P cements such as MgNH₄PO₄, formed via reaction between acid part (ammonia salts) and base part (MgO or Mg(OH)₂), struvite, newberyite, or cattiite; Sr-P cements (SPCs), such as Ca-P cements doped with strontium, or Mg-P cements doped with strontium; calcium sulfate cements, such as calcium sulfate hemihydrate (CSH) and calcium sulfate dehydrate (CSD); poly methyl methacrylate (PMMA) cements; and polyacrylic acid (PAA) cements.

US5374427A discloses materials which are especially suited to form implantable active substances depots. The depots according to US5374427A comprise a number of constituents including, *inter alia,* a foam-generating agent. As a foam-forming constituent, all carbonates and/or hydrogen carbonates are mentioned. Also mentioned are metal hydrides, in particular, sodium boron hydride, which cause a foaming of the hardening cement with protons (water or acids), accompanied by hydrogen development.

Existing porous cement compositions, which typically use NaCl as a porogen, ordinarily require high temperature sintering steps from granulation to leaching of NaCl. Even if high temperature sintering is not required, the known methods typically require a few steps from granulation to leaching. Furthermore, these processes are not continuous because of the steps involved in creating the porosity. The known methods are specifically tailored toward the fabrication of granules instead of an injectable product, despite the appeal of an injectable macroporous cement composition. The known porous cement products are also not radio-opaque. Thus, the methods and compositions disclosed herein include many advancements and improvements over currently known methods and cement compositions.

In accordance with the present disclosure, the cements described herein use a metal comprising magnesium as a porogen, which instantaneously creates porosity and is biocompatible. This porogen does not require subsequent leaching in water, unlike cements that use NaCl as a porogen. Furthermore, because of the instantaneous creation of pores, the process can be truly termed a single-step process. Either continuous or batch processes can be used to make these cements, and the cements can be made into injectable compositions. The ability to make an injectable, macroporous cement composition in real-time (that is, within a matter of a few minutes) is a significant advantage over existing methods and cement compositions. Furthermore, the cement compositions described herein generate little or no exothermicity during hardening. This alleviates concerns of possible damage to surrounding tissue while curing. The compositions are also pH-balanced.

The particular porogen used in this disclosure is one that reacts with the liquid component to give off a gas. The gas bubbles produced from this reaction do not disappear prior to the gaseous solution hardening. Rather, as the gas bubbles attempt to escape, they are trapped by the hardened of the solution, thereby creating pores in the hardened cement mass when the gaseous solution sets. The porogen is one that reacts with water to give off hydrogen gas. However, other gases can be generated to create porosity.

In particular embodiments, the porogen is magnesium metal particles. Moreover, many magnesium alloys, such asalloys of magnesium and yttrium, magnesium and zinc, magnesium and aluminum, or magnesium and other rare-earth metals, can produce hydrogen gas upon mixture with water and thus are suitable for use as a porogen in the methods and compositions described herein. Magnesium is advantageous over other known porogens because magnesium corrodes quickly and forms bubbles upon mixing with water quickly. Magnesium also has a cytotoxicity better suited for cement applications than other metals.

Magnesium is known to be highly prone to corrosion and is readily available in powder form. Magnesium is the fourth most abundant element in the mammalian body, and most of it is stored in the skeleton. When magnesium comes into contact with water, it evolves hydrogen gas. Thus, when magnesium particles are mixed with a powder component and an aqueous liquid, the evolution of hydrogen bubbles creates instant porosities of up to about 1000 µm. Without limitation, the pores created by evolving hydrogen gas range from about 100 µm to about 1 mm in size. By carefully changing the power-to-liquid ratio, it is possible to inject this free-flowing mass as a porous product containing macroporosity.

The magnesium used as a porogen is preferably in the form of spherical particles in a very fine magnesium mesh. The reason spherical particles are preferred is because they have a higher surface area, and therefore greater reactivity. The greater reactivity allows for more hydrogen bubbles to form from the reaction between magnesium and water. Additionally, the shape of the particles determines the shape of the pores. Therefore, spherical magnesium particles produce spherical pores in the resulting cement. If differently shaped pores are desired for a particular cement application, the shape of the porogen particles can be chosen accordingly. The magnesium particles can also be sieved into different size fractions in order to control the porosity of the resulting cement.

A typical bone cement is made by mixing a basic powder component with an acidic liquid component, referred to as a setting solution, in order to form a paste, resulting from an acid-base reaction, that sets into a hardened mass. Many variations of this method have been developed. For instance, as described in WO 2013/116457, the paste can be subjected to microwave radiation prior to hardening and crushed up, in order to form dry powder, also referred to as post-microwaved powder (PMP), that is then mixed with a second setting solution to form a radiation-assisted paste that sets into a hardened mass. Silica can be included in the second setting solution in order to improve the strength of the resulting cement. In a typical example, the second setting solution can contain about 40% silica suspended in water. Also described in WO 2013/116457, a setting solution can include a biopolymer, such as chitosan, in order to improve the strength of the resulting cement. Alternatively, a polysaccharide, such as alginate, can be included in a setting solution in order to improve the handling of the cement. Alginate dissolves in the body quickly, and is very hydrophilic. The inclusion of alginate tends to improve the handling properties of the resulting cement. Furthermore, the addition of magnesium oxide can improve the strength of the resulting cement. These methods of producing a bone cement are referred to for exemplary purposes herein because they are especially conducive to preparing an injectable, macroporous composition in a matter of a few minutes, though any other method of producing a cement composition that involves the use of a liquid may be used. In preferred embodiments, the liquid is an aqueous solution. This is because the chemistry of forming hydrogen gas from a reaction between a porogen and water is especially useful for creating injectable, macroporous compositions.

In any method of making the macroporous cement compositions, the porogen particles are added either to the powder to be mixed with a liquid (i.e., a setting solution or second setting solution), to a pre-mixed paste that ultimately sets into a hardened mass, to the liquid immediately prior to mixing with the powder component, or to the powder and liquid simultaneously. Upon contact with the liquid, the porogen begins to react, for instance with water, to form gas bubbles, such as hydrogen gas bubbles. The gas bubbles try to escape from the gaseous solution while the solution sets, but the gaseous solution sets into a hardened mass before the gas bubbles can fully escape. In this manner, the gas bubbles become pores in the resulting hardened mass, thereby creating a porous cement mass. Given the size of the pores possible using this method, the cement mass is a macroporous cement mass. Those skilled in the art will understand that the injectable cement can be manipulated, shaped, or extruded while setting in order to form a desired shape, for example to fit into a desired mold or position. The gaseous solution can be formed into macroporous blocks or putties before hardening.

The setting time for a cement composition using magnesium can vary widely, and can be customized. In certain embodiments, the setting time is between about 30 seconds and 1 minute. This period of time allows for hydrogen bubbles to come to the surface of the composition. In addition, the pore attributes can be altered and customized for a particular application. For instance, by incorporating alloys of magnesium, such as with Al, Zn, or rare-earths, the degradation rate of the metallic additions, and therefore the hydrogen evolution rates, is changed. This, in turn, affects the amount and size of pores created before the composition sets into hardened granules.

Given that magnesium is a brittle ceramic, there is a possibility of the granules crumbling if too many pores or too large of pores are generated. To avoid this, the cement composition should be made with sufficient strength. Thus, instead of granulating batches, a continuous process of injection of the cement pase involving extrusion followed by subsequent chopping can be a method for production. A variation of the process may involve injecting the high porosity mixture directly into an orthopedic injury or cavity site. During the process of extrusion/injection, substantially all the magnesium powder particles totally convert into hydrogen gas bubbles. By carefully choosing the size and distribution of magnesium particles, the ranges of pore sizes can be easily controlled.

The powder-to-liquid ratio determines the setting time and hardnening of the cement composition. Thus, the setting time of a macroporous cement composition can be adjusted by changing the powder-to-liquid ratio of the cement. In this manner, the cement compositions are customizable for a desired application. For instance, the relevant practitioner would likely want the setting time of a cement to be used for filling a cavity in a tooth to be somewhat quicker than the setting time of a cement to be used in an orthopedic surgery application.

If a reduced particle size of a particular cement composition is desired for a certain application, such particle reduction can be accomplished by using, for example, an agate pestle and mortar, a ball mill, a roller mill, a centrifugal-impact mill and sieve, a cutter mill, an attrition mill, a chaser mill, a fluid-energy mill, and/or a centrifugal-impact pulverizer. Particle size reduction may be desired for treating bone defects through a method that involves breaking up the hardened cement into pellets and filling a hole or cavity in a bone with the pellets.

The cement compositions described herein can be used to deliver one or more agents. Suitable agents include, but are not limited to: drugs, such as bisphosphonates, parathyroid hormones, antiseptics, anti-inflammatories, anti-infectives, antibiotics, antifungals, bone resorption inhibitors, antivirals, analgesics, chemotherapeutic agents, anticoagulants, antihistamines, antineoplastic agents, steroids, and vitamins; proteins, including growth factors, such as the bone morphogenetic proteins BMP1, BMP2, BMP3, BMP4, BMP5, BMP6, BMP7, BMP8a, BMP8b, BMP10, or BMP15; cells, such as mesenchymal stem cells; nucleic acids, such as microRNAs, antisense microRNAs, siRNAs, shRNAs, cDNA, and aptamers; drug delivery vehicles (whether loaded with drugs or not), such as liposomal formulations or carbon nanotubes; diagnostic agents; materials designed to release ions, such as silver ions in the form of a colloidal silver; bioactive glass; and combinations thereof. The agents can be incorporated into the walls of the pores in the macroporous cement, and are released upon degradation of the cement.

The cement compositions disclosed herein are particularly useful for drug delivery. The macroporous cements described herein have increased surface area from which drugs can be delivered. To load a drug in the cement, the drug can be dissolved in a gaseous solution prior to setting into a macroporous cement mass, or a macroporous cement composition (before or after it sets) can be soaked in a solution or suspension comprising the drug, before the composition is injected or placed into or onto an anatomical location. The drug can then be released into the subject from the cement matrix. Embodiments resulting in sustained release of drugs are also possible. The rate of release of a drug depends on the degradability of the cement. Thus, the degradability of the cement composition can be controlled (for instance, by changing the powder and liquid components in order to produce a different phase) in order to control the rate at which a drug is released from the cement composition. Alternatively, sustained release can be accomplished by coating the cement matrix with polymers including PLA/PGA, polyacrylic acid, hydroxyl methylcellulose, and/or chitosan. As yet another alternative, the drugs may be in the form of a lipophilic ester, such as an acyl derivative (for instance, an acetyl ester), so as to allow release of the drugs from the cement over a prolonged period of time as a result of esterase activity in the physiological fluids contacting the cement in the body.

The delivery of drugs from the cement is dictated by the composition of the cement. The release of drugs depends upon the rate of degradation of the composition. Thus, cement compositions that degrade faster will release drugs at a quicker rate. For instance, DCPD cement is somewhat faster at degrading, and therefore faster at releasing drugs, than monetite cement.

In one particular embodiment, the cement composition is useful for treating or preventing an infection, and the composition includes at least one antibiotic. Suitable antibiotics include, but are not limited to, penicillins, tetracyclines, glycylcyclines, macrolides, sulfonamides, cephalosporins, monobactams, carbapenems, aminoglycosides, quinolones, oxazolidinones, and combinations thereof. In certain non-limiting examples, the composition includes at least one antibiotic selected from the group consisting of clindamycin, tigecycline, vancomycin, ciprofloxacin, ofloxacin, sulfamethoxazole, trimethoprim/sulfamethoxazole, amoxicillin, penicillin V, penicillin G, procaine penicillin, benzathine penicillin, carbencillin, mezlocillin, ampicillin, piperacillin, bacampicillin, tiearcillin, ticarcillin, piperacillin/tazobactam, aztreonam, cefotetan, loracarbef, mefoxin, merrem, levofloxacin, lomefioxacin, primaxim, cycloserine, kanamycin, dicloxacillin, demeclocycline, minocycline, doxycycline, oxytetracycline, tobramycin, gentamicin, neomycin, amikacin, craramyein, nebcin, erythromycin/sulfisoxazole, netromycin, streptomycin, tobramycin, cefotaxime, cefuroxime, cefazoline, ceffibuten, ceffizoxime, cefaclor, cefopoerazone, cefprozil, cefadroxil monohydrate, ceftazidime, trimethoprim/sulfamethoxazole, cephalexin, cefazolin, cefamandole nafate, cefepime, cefonicid, sulfadiazine, norfloxacin, enoxacin, cefdinir, seromycin, ceftriaxone, cefixime, ceftazidime, clarithromycin, dirithromycin, methenamine, ethionamide, trovafioxacin, sparfloxacin, interfon-α, indinavir, ganciclovir, foscamet, lamivudine, famciclovir, rimantadine, zalcitabine, interferon-β, indinavir, ganciclovir, saquinavir, ritonavir, ribavirin, erythromycin, troleandomycin, azithromycin, eliiidamycin, colistin, amphotericin B, flucytosine, fluconazole, griseofulvin, grepafloxacin, ultramicrosize griseofulvin, terbinafine, ketoconazole, clotrimazole, dapsone, delavirdine, ziduvudine, amantadine, palivizumab, valacyclovir, didanosine, nelfinavir, nevirapine, ribavirin, cidofovir, pyrimethamine, metronidazole, furazolidone, atovaquone, stavudine, lamiduvine, acyclovir, mionazole, nystatin, itraconazole, chloroquine, pyrimethamine, mefloquine, hydroxychloroquine, capreomycin, permethrin, crotamiton, lindane, fluoro-uracil, ethambutol, rifabutin, isoniazid, aminosalicyclic acid, rifapentine, pyrazinamide, coenzoyl peroxide, chlorhexidine gluconate, sodium oxychlorosene, benzoyl peroxide, rifampin, rifampin/isoniazid, rifampin/isoniazid/pyrazinamide, nitrofurantoin, linezolid, nitrofurantoin, fosfomycin, nalidixic acid, atropine, oxytetracycline/sulfamethizole/phenazopyridine, mupirocin, chloramphenical, neomycin/polymyxin, tfimetorpim/polythyxin, tobramycin/dexamethasone, vidatabine, ciprofloxacin, ofioxacin, sulfacetamide, mupirocin, povidoneodine, gentamicin, nystatin, chloramphenicol, bacitracin, sulconazole, terbinafine, tetrachlorosalicylanilide, metronidazole, metromdazole, ciclopiroxolamine, clotrimazole, clotrimazole/betamethasone, butenafine, clotrimazole, nattifine, oxiconazole, selenium, econazole, penciclovir, or a pharmaceutically acceptable salt thereof.

In another particular embodiment, the cement composition is particularly useful for the healing of a fracture, and the composition includes at least one of BMP2 or BMP7. In another particular embodiment, the cement composition is useful for the prevention or management of osteoporosis, and the composition includes a drug selected from the group consisting of risedronate, etidronate, alendronate, teriparatide, strontium ranelate, raloxifene, denosumab, and calcitonin.

Various additives, other than drugs, may be included in any of the cement compositions described herein to adjust their properties and the properties of the hardened cements produced. Examples of suitable additives include, but are not limited to, proteins, osteoinductive and/or osteoconductive materials, X-ray opacifying agents such as strontium phosphate or strontium oxide, supporting or strengthening filler materials, crystal growth adjusters, viscosity modifiers, additional poreforming agents, color change agents, immersing liquids, carboxylates, carboxylic acids, α-hydroxyl acids, metallic ions, or mixtures thereof. Other suitable additives include substances that adjust setting times (such as pyrophosphates or sulfates), increase injectability or cohesion (such as hydrophobic polymers like collagen), or alter swelling time.

In addition to delivering drugs or other agents, the macroporous cement compositions described herein can be used for any bone cement application. By way of non-limiting examples, the macroporous cement compositions can be: used as a bone filler in areas of the skeleton where bone may be deficient; used to fill, augment, and/or reconstruct maxillofacial osseous bone defects, including for periodontal, oral, and cranio-maxillofacial application; packed gently into bony voids or gaps of the skeletal system (i.e., the extremities, pelvis, and spine), including for use in postero-lateral spinal fusion or vertebral augmentation procedures with or without appropriate stabilizing hardware; used to fill defects which may be surgically creasted osseous defects or osseous defects created from traumatic injury to the bone; used to heal a bone fracture; used as a filling material in teeth; coated on titanium implants; and added to calcium sulfate dehydrate (CSD) cements in order to significantly increase various properties of the CSD cements. The macroporous cement compositions can provide a bone void filler that resorbs and is replaced by bone during the healing process.

The macroporous cement compositions can be supplied to the user in a variety of forms, including as a powder or powder mixture which is later mixed with a solvent to make a slurry or putty, or as a pre-mixed putty which may contain a non-aqueous extender such as, but not limited to, glycerine and/or propylene glycol. The pre-mixed putty would allow the cement to set upon contact with water. In such embodiments, the porogen can be added with the water in order to introduce porosity. Also, any of the cement compositions described herein can be delivered via injection. That is, a cement composition can be transferred to a syringe for injection into a mammalian body prior to fully hardening. Furthermore, the cement compositions may be supplied with, or in, the instrumentation which is used to introduce the cement into the body. Examples of such instrumentation include, but are not limited to, a syringe, a percutaneous device, a cannula, a biocompatible packet, a dentula, a reamer, a file, or other forms which will be apparent to those of ordinary skill in the art. It is also understood that the cement compositions described herein could be delivered into the body in such a form as to be converted by bodily processes into the macroporous cement compositions disclosed herein.

The cement compositions disclosed herein may also be made available to the user (including practitioners such as surgeons, veterinarians, or dentists) via a kit containing one or more key components. A non-limiting example of such a kit comprises a dry homogeneous powder (composed of the powder component and magnesium particles) in one container, and the liquid component in another container, where the containers may or may not be present in a combined configuration. Many other kits are possible, such as kits comprising a pre-mixed putty instead of the powder and setting solution, and kits including a syringe or multiple syringes for injecting a macroporous cement composition formed from the components of such kit. The kits typically further include instructions for using the components of the kit to practice the subject methods. The instructions for practicing the subject methods are generally recorded on a suitable recording medium. For example, the instructions may be present in the kits as a package insert or in the labeling of the container of the kit or components thereof. As an alternative, the instructions are present as an electronic storage data file present on a suitable computer readable storage medium, such as a CD-ROM, flash drive, or diskette. As another alternative, the actual instructions are not present in the kit, but means for obtaining the instructions from a remote source, such as via the internet, are provided. An example is a kit that includes a web address where the instructions can be viewed and/or from which the instructions can be downloaded. As with the instructions, this means for obtaining the instructions is recorded on a suitable substrate.

The components to form a macroporous cement composition as described herein may be present as a packaged element. The cements are generally provided or employed in a sterilized condition. Sterilization may be accomplished by several methods such as radiation sterilization (e.g., gamma-ray radiation), dry heat sterilization, or chemical cold sterilization.

### EXAMPLES

### Example 1

Pure magnesium powder (-60 mesh) was weighed out at 0.05 grams and added into a mortar bowl. Then, 0.05 grams of magnesium oxide with 2 grams of the microwave-irradiated CaP cement powder used PCT Publication WO 2013/116457 were added to the mortar bowl. The powders were mixed thoroughly using an agate pestle to provide a uniform reaction. Finally, 0.5 ml of water and 1.5 ml of silica sol were added to the mixture and manually mixed using an agate pestle. Use of the agate pestle was stopped after all the powder had come into contact with the water. This allowed for the reaction to occur and for hydrogen bubbles to escape, creating pores throughout the mass without any trace of magnesium left. After the reaction took place, the macroporous granules reached optimum strength in approximately two hours. The granules were then placed into a water bath. After twenty-four hours, the cement remained intact and retained its strength without getting crumbled into powder.

### Example 2

Post-microwaved powder (PMP) was synthesized from calcium hydroxide (98% Ca(OH)₂ Fisher, Fair Lawn, NJ as the source of calcium along with the setting solution). The PMP was comprised of 18.4 g of Ca(OH)₂ and 4.6 g of Mg(OH)₂. This ratio can be altered in order to make various batch sizes of different yields. The setting solution was prepared by combining sodium bicarbonate (NaHCO₃, Spectrum, CAS: 144-55-8), de-ionized water, and phosphoric acid (H₃PO₄, Spectrum, CAS: 7664-38-2). To yield 180 ml of complete setting solution, the setting solution consisted of 24 g sodium bicarbonate, 24 ml DI water, and 156 ml phosphoric acid, respectively. PMP was prepared by manually mixing calcium hydroxide with the setting solution using a mortar and pestle. For example, a batch of PMP was made by dispersing 18.4 g of calcium hydroxide and 4.6 g of magnesium hydroxide in 30 ml of setting solution, and completely mixing the powder in the setting solution through mixing for one minute. The resulting paste was then microwaved for nine minutes with 120 watts power to dehydrate the cement in order to stop the reaction. The resulting hard paste was crushed with a mortar and pestle and finally ground to create a fine powder. This PMP powder was used in the subsequent examples to create macroporous compositions.

### Example 3

The as-synthesized PMP was mixed with spherical Mg particles (<325 mesh; on the average ~ 45 µm in diameter) in the compositions detailed in Table 1, below.

**Table 1 - PMP Compositions with Mg Particles**

| Mg powder (g) | MgO (g) | PMP (g) | H₂O (ml) |
|---|---|---|---|
| 0.05 | 0.05 | 2 | 1 |
| 0.05 | 0.1 | 2 | 1.1 |

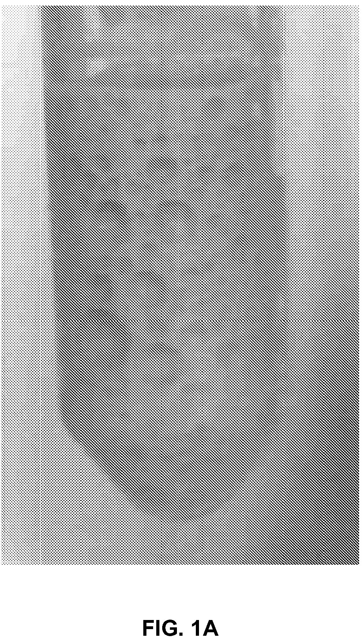

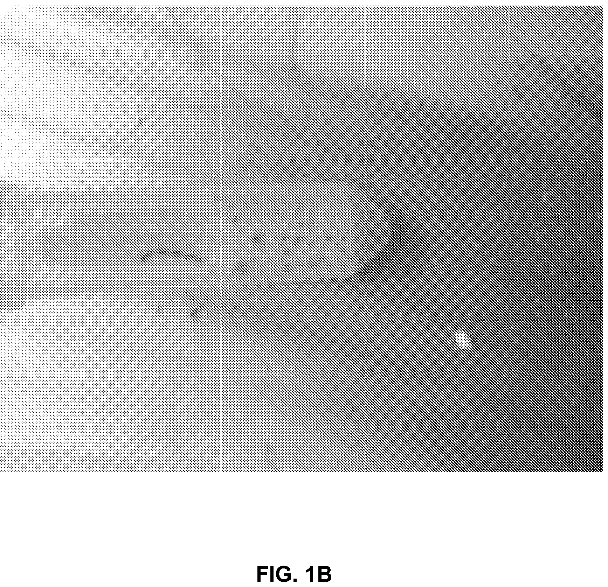

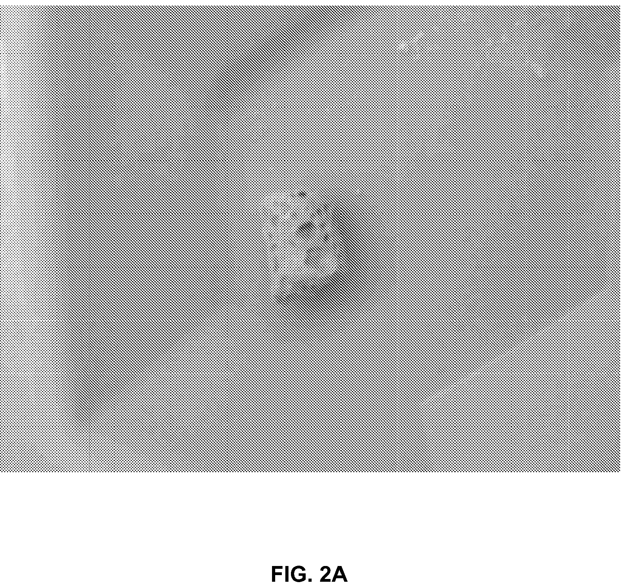

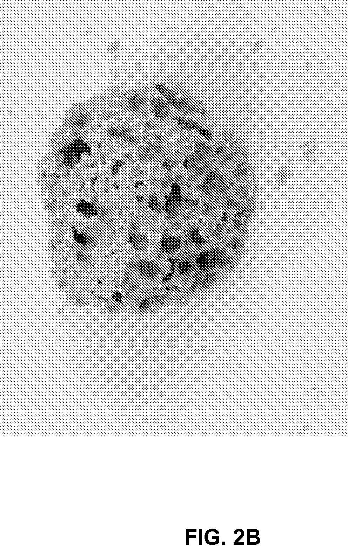

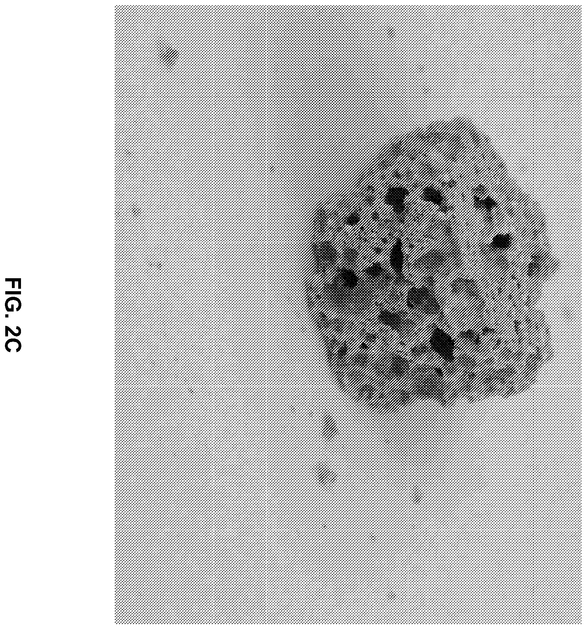

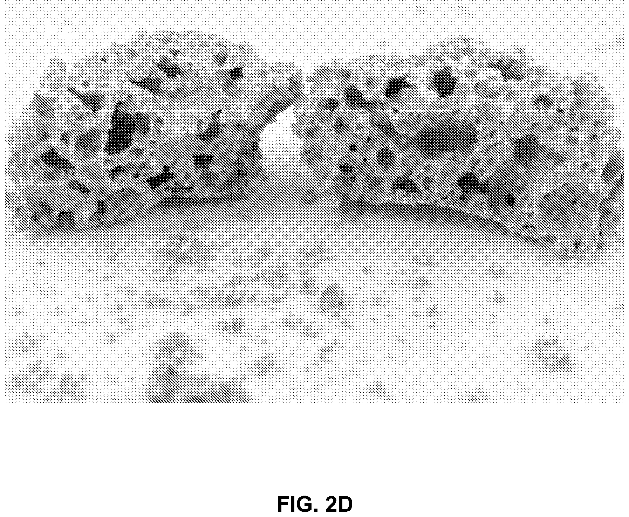

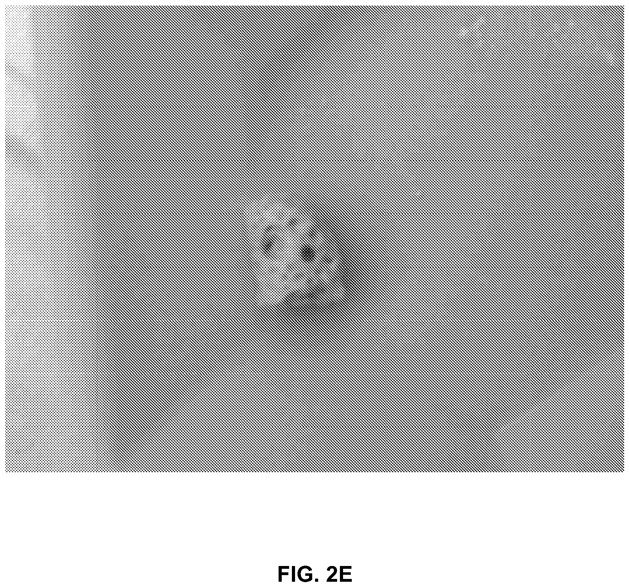

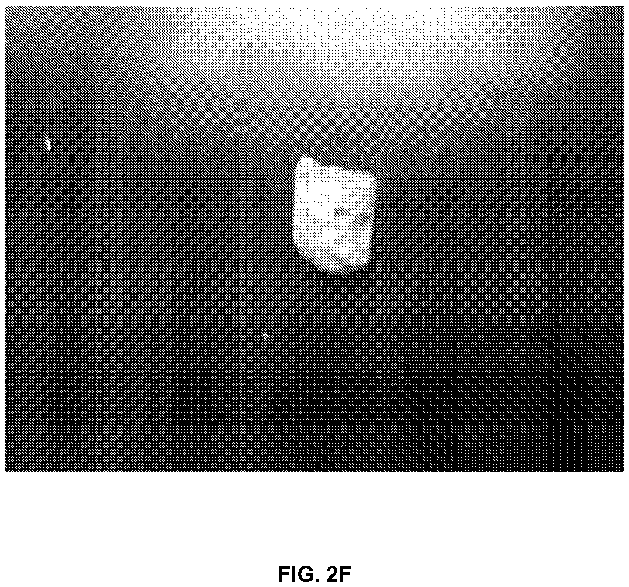

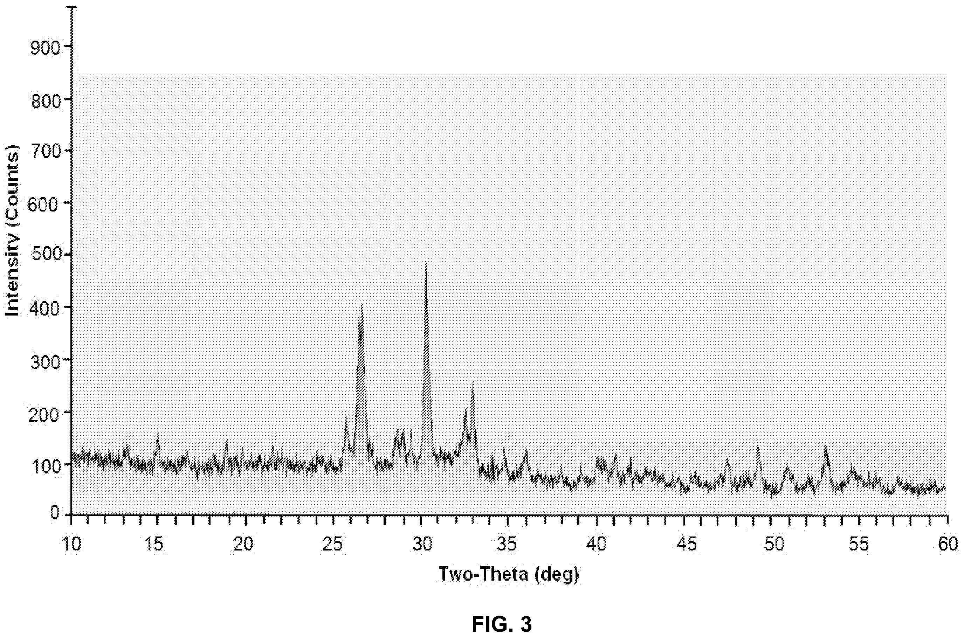

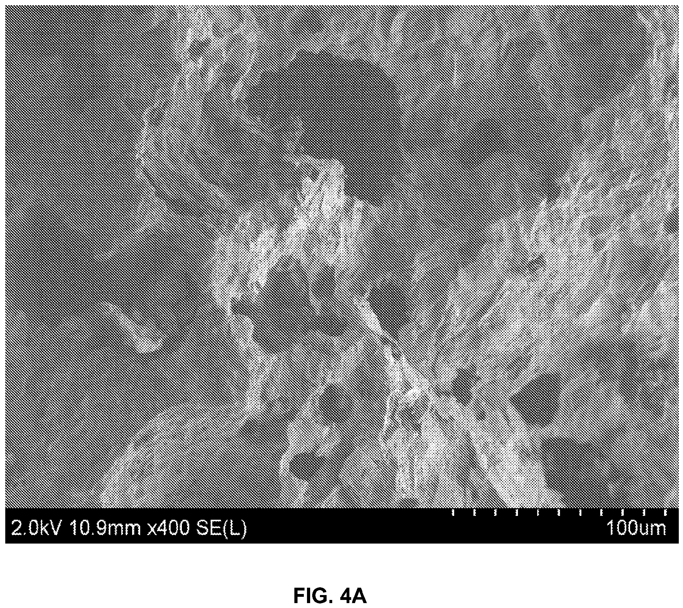

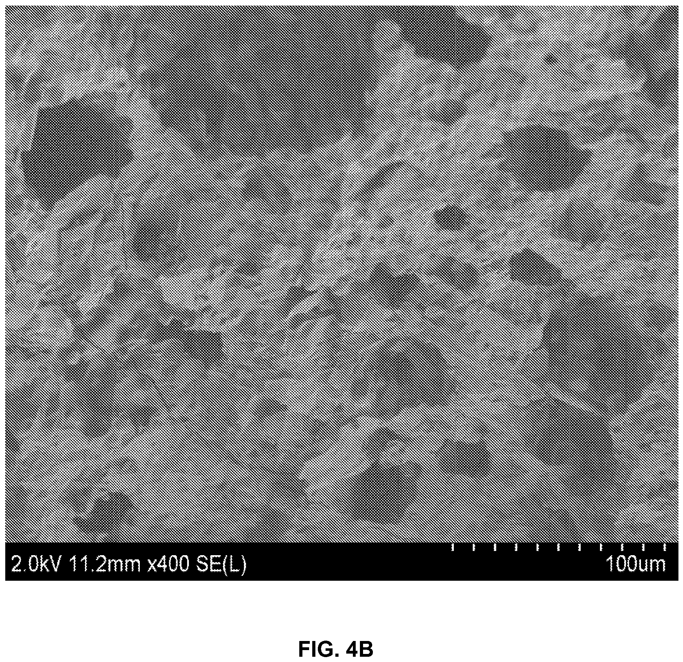

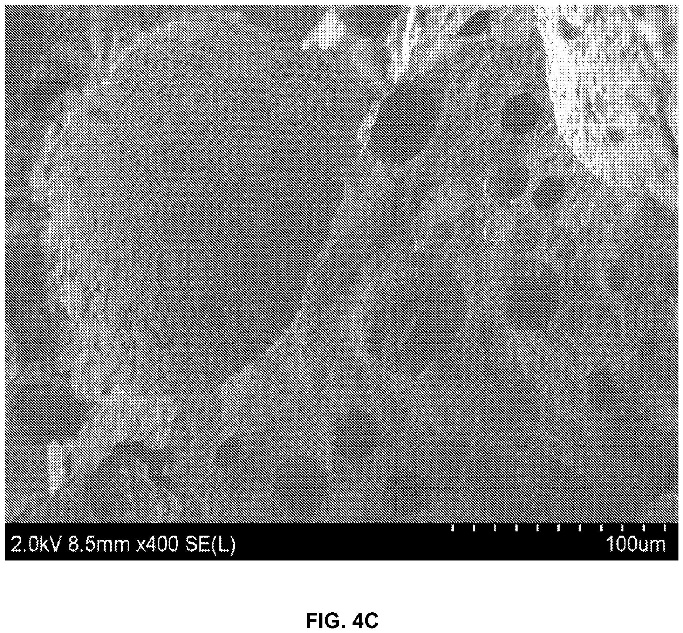

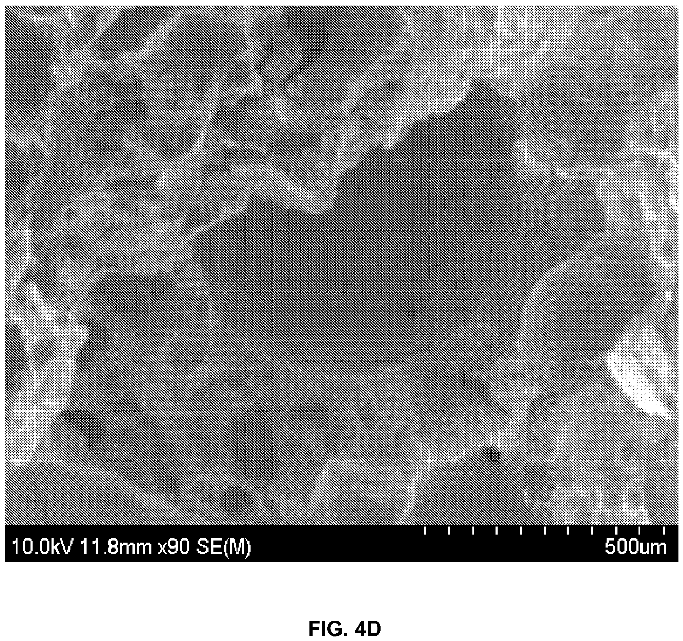

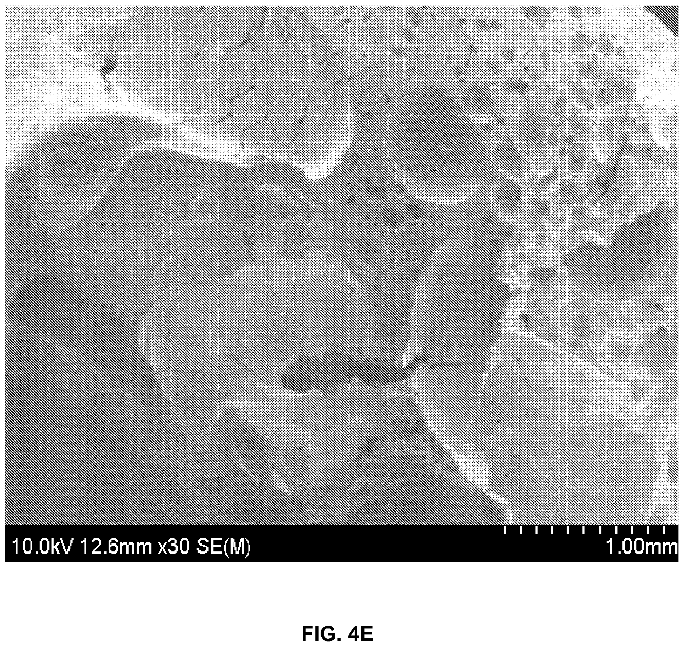

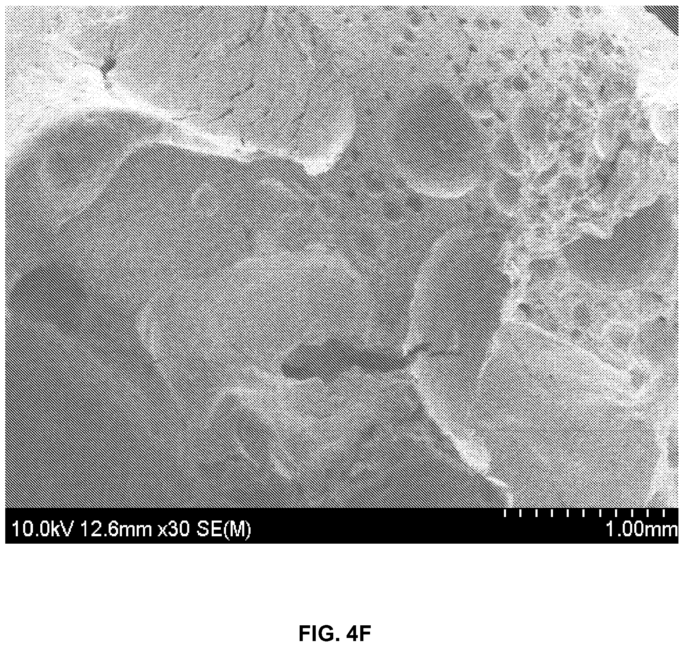

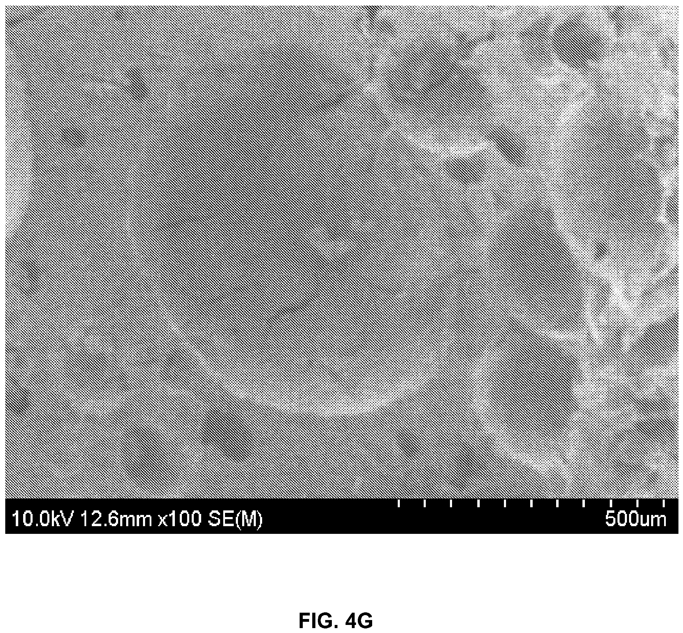

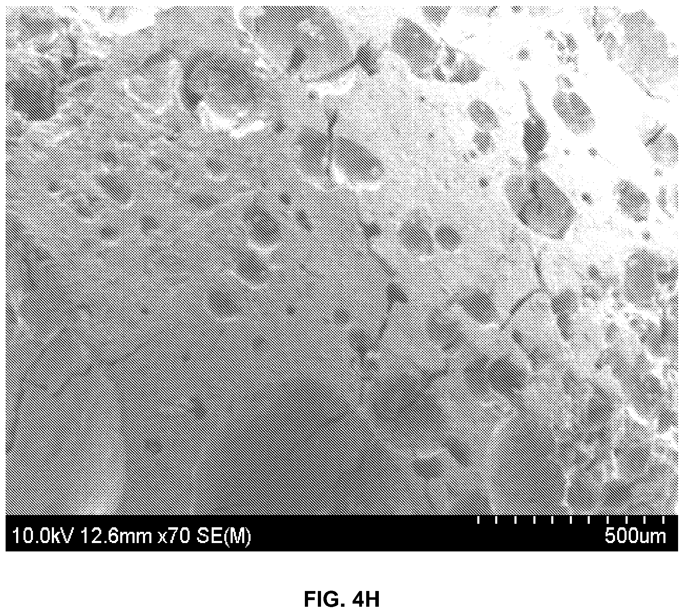

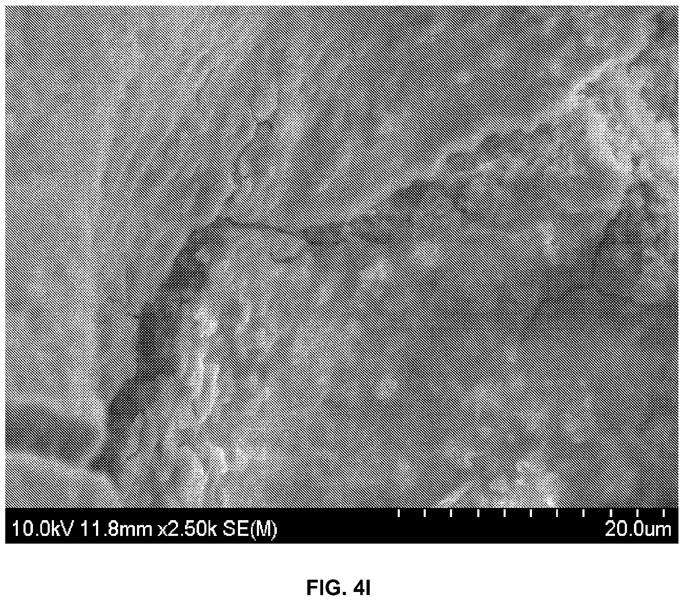

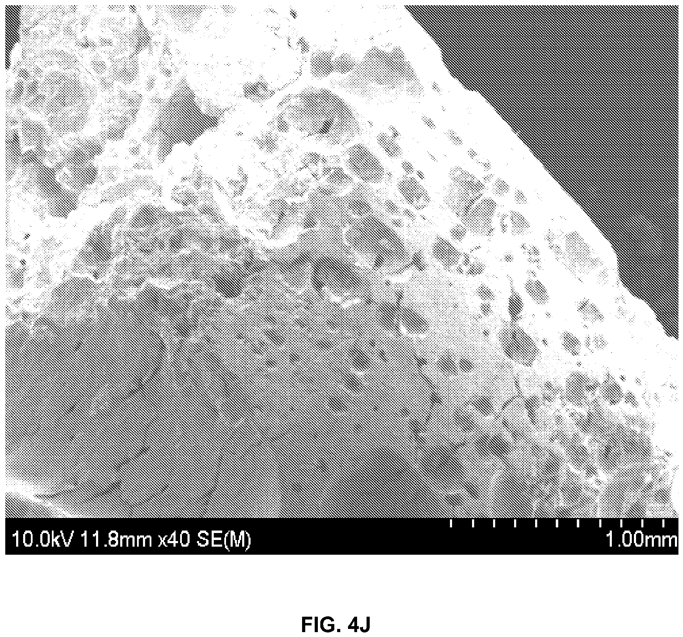

Both of the above compositions were gently mixed in a mortar and pestle. When the Mg particles were dissolved, there was concomitant evolution of H₂ gas. Within 2-3 minutes, the compositions began to harden while creating macropores approximately 500 µm in diameter. The formation of pores from hydrogen gas evolution can be seen in the photographs in **FIGS. 1A-1B****.** These masses could be injected into a thin tube with pores still evolving *in situ,* prior to hardening. After complete hardening, the granules had sufficient handling strength. Photographs of the resulting hardened granules are shown in **FIGS. 2A-2F****.** An X-ray powder diffraction pattern of a macroporous composition made by this process is shown in **FIG. 3****.** **FIGS 4A-4J** are SEM images of a hardened granule from this process. The SEM images show the different sizes of the pores formed in the hardened granules.

### Example 4

The procedure of Example 3 was repeated with a slightly different composition, as noted in Table 2, below.

**Table 2 -PMP Composition with Mg Particles and Higher Volume of Water**

| Mg powder (g) | PMP (g) | H₂O (ml) |
|---|---|---|
| 0.1 | 2 | 2 |

This composition was made the same way as described in above, and pore generation took place *in situ,* as described in the previous example. With the addition of more water, the setting time increased. However, the strength was reduced due to the more time available for the macropores to grow.

### Example 5

To enhance the strength of the macroporous samples, both MgO and colloidal silica (HS-40, WR Grace) were incorporated into the sample. This composition is detailed in Table 3, below.

**Table 3 - PMP Composition with Colloidal Silica and MgO**

| Mg powder (g) | MgO (g) | PMP (g) | H₂O (ml) | HS-40 (ml) |
|---|---|---|---|---|
| 0.05 | 0.05 | 2 | 0.5 | 1.5 |

This material had all the attributes of the previous example in terms of hardening and creation of macropores *in situ.* Additionally, this composition had high strength in spite of the presence of large macropores. This cement set very well and rather quickly, producing great porosity and strength.

### Example 6

Another composition with enhanced strength was obtained by mixing PMP with magnesium phosphate in the ratio of 1:1 with HS-40 (colloidal silica suspended in water) as the liquid and pH buffered at 4.0. This composition is detailed in Table 4, below.

**Table 4 - PMP Composition with MgP and Colloidal Silica**

| Mg powder (g) | MgO (g) | PMP (g) | MgP (g) | HS-40 (ml) | pH 4 buffer (ml) |
|---|---|---|---|---|---|
| 0.05 | 0.05 | 1 | 1 | 1.5 | 0.5 |

Acidic medium is important in certain situations. By mixing PMP and MgP in a 1:1 ratio, all the attributes of the previous compositions could be retained.

### Example 7

The composition from Example 6 was further modified by adding alginate to it. The alginate enhanced the handling strength as well as cell growth. This composition is detailed in Table 5, below.

**Table 5 - PMP Composition with Alginate**

| Mg powder (g) | MgO (g) | PMP (g) | HS-40 (ml) | pH 4 buffer (ml) | Alginate (g) |
|---|---|---|---|---|---|
| 0.05 | 0.05 | 10 | 7.5 | 2.5 | 1 |

The sample solidified almost immediately, in a time period ranging from about 30 seconds to about 2 minutes. The composition was placed into DI H₂O after a few minutes and retained its structure. The resulting cement had great handling properties. The strength of cement was very high.

## Claims

1. A method for making a cement composition for orthopedic or dental applications comprising:
mixing a powder component with a liquid component and a metal to form a gaseous solution, wherein the metal comprises magnesium and reacts with water in the liquid component to produce hydrogen gas bubbles in the gaseous solution; and
allowing the gaseous solution to set into a hardened porous mass, wherein the hydrogen gas bubbles create pores in the hardened mass;
wherein the liquid component comprises silica; and
wherein the powder component is a post-microwaved powder prepared by mixing a powder with a setting solution to form a paste, and irradiating the paste with microwaves to form the post-microwaved powder.

2. The method of claim 1, wherein the metal is in the form of spherical particles.

3. The method of claim 1, wherein the metal is present in an alloy with one or more of Al, Zn, or a rare-earth metal.

4. The method of claim 1, wherein at least one of alginate or chitosan is mixed with the powder component, the metal, and the liquid component.

5. The method of claim 1, wherein the hardening of the gaseous solution occurs simultaneously with the production of the hydrogen gas bubbles in the gaseous solution.

6. The method of claim 1, wherein the composition is extruded through a syringe while the pores are forming in the gaseous solution from the release of the hydrogen gas bubbles.

7. A macroporous cement composition for orthopedic or dental applications comprising pores made from a hydrogen-evolving process that occurs in a solution containing a mixture of a post-microwaved powder and a liquid comprising silica, wherein the pores range from about 100 µm to about 1 mm in size, and wherein the macroporous cement composition comprises a magnesium phosphate that has been irradiated by microwaves, the macroporous cement composition being injectable during the hydrogen-evolving process.

8. The macroporous cement composition of claim 7, wherein the hydrogen-evolving process is a reaction between magnesium particles and water present in a liquid.

9. The macroporous cement composition of claim 7, wherein the macroporous cement composition comprises a biopolymer or polysaccharide.

10. The macroporous cement composition of claim 7, further comprising drug molecules in the pores.

## Patentansprüche

1. Verfahren zum Herstellen einer Zementzusammensetzung für orthopädische oder dentale Anwendungen, mit:
Mischen einer Pulverkomponente mit einer Flüssigkomponente und einem Metall zum Ausbilden einer Gaslösung, wobei das Metall Magnesium aufweist und mit Wasser in der Flüssigkomponente zum Erzeugen von Wasserstoffgasblasen in der Gaslösung reagiert; und
Ermöglichen, dass die Gaslösung zu einer gehärteten porösen Masse erstarrt, wobei die Wasserstoffgasblasen Poren in der gehärteten Masse erzeugen;
bei dem die Flüssigkomponente Siliciumdioxid aufweist und
bei dem die Pulverkomponente ein Pulver nach einer Mikrowellenbestrahlung ist, das durch Mischen eines Pulvers mit einer Erstarrungslösung zum Bilden einer Paste und Bestrahlen der Paste mit Mikrowellen zum Ausbilden des Pulvers nach einer Mikrowellenbestrahlung bereitgestellt wird.

2. Verfahren nach Anspruch 1, bei dem das Metall in Form von kugelförmigen Partikeln vorliegt.

3. Verfahren nach Anspruch 1, bei dem das Metall in einer Legierung mit einem oder mehreren von Al, Zn oder einem Seltenerdmetall vorhanden ist.

4. Verfahren nach Anspruch 1, bei dem mindestens Alginat oder Chitosan mit der Pulverkomponente, dem Metall und der Flüssigkomponente vermischt wird.

5. Verfahren nach Anspruch 1, bei dem das Härten der Gaslösung gleichzeitig mit der Produktion der Wasserstoffgasblasen in der Gaslösung auftritt.

6. Verfahren nach Anspruch 1, bei dem die Zusammensetzung durch eine Spritze extrudiert wird, während sich die Poren aufgrund der Freisetzung der Wasserstoffgasblasen in der Gaslösung bilden.

7. Makroporöse Zementzusammensetzung für orthopädische oder dentale Anwendungen, mit Poren, die durch einen Wasserstofffreisetzungsprozess, der in einer Lösung, die ein Gemisch aus einem Pulver nach einer Mikrowellenbestrahlung und einer Flüssigkeit mit Siliciumdioxid enthält, auftritt, hergestellt worden sind, wobei die Porengröße zwischen etwa 100 µm und etwa 1 mm liegt und die makroporöse Zementzusammensetzung ein Magnesiumphosphat aufweist, das mit Mikrowellen bestrahlt worden ist, wobei die makroporöse Zementzusammensetzung während des Wasserstofffreisetzungsprozesses einspritzbar ist.

8. Makroporöse Zementzusammensetzung nach Anspruch 7, bei dem der Wasserstofffreisetzungsprozess eine Reaktion zwischen Magnesiumpartikeln und in einer Flüssigkeit vorhandenem Wasser ist.

9. Makroporöse Zementzusammensetzung nach Anspruch 7, bei dem die makroporöse Zementzusammensetzung ein Biopolymer oder ein Polysaccharid aufweist.

10. Makroporöse Zementzusammensetzung nach Anspruch 7, ferner mit Medikamentenmolekülen in den Poren.

## Revendications

1. Procédé de fabrication d'une composition de ciment pour des applications orthopédiques ou dentaires comprenant :
le mélange d'un composant en poudre avec un composant liquide et un métal pour former une solution gazeuse, dans lequel le métal comprend du magnésium et réagit avec de l'eau dans le composant liquide pour produire des bulles d'hydrogène gazeux dans la solution gazeuse ; et
le fait de laisser la solution gazeuse se figer en une masse poreuse durcie, dans lequel les bulles d'hydrogène gazeux créent des pores dans la masse durcie ;
dans lequel le composant liquide comprend de la silice ; et
dans lequel le composant en poudre est une poudre post-traitement aux micro-ondes, préparée par mélange d'une poudre avec une solution de figeage pour former une pâte, et irradiation de la pâte avec des micro-ondes pour former la poudre post-traitement aux micro-ondes.

2. Procédé selon la revendication 1, dans lequel le métal est sous la forme de particules sphériques.

3. Procédé selon la revendication 1, dans lequel le métal est présent dans un alliage avec un ou plusieurs de Al, Zn, ou un métal de terres rares.

4. Procédé selon la revendication 1, dans lequel au moins l'un de l'alginate ou du chitosan est mélangé avec le composant en poudre, le métal, et le composant liquide.

5. Procédé selon la revendication 1, dans lequel le durcissement de la solution gazeuse survient simultanément à la production des bulles d'hydrogène gazeux dans la solution gazeuse.

6. Procédé selon la revendication 1, dans lequel la composition est extrudée à travers une seringue tandis que les pores sont formés dans la solution gazeuse à partir de la libération des bulles d'hydrogène gazeux.

7. Composition de ciment macroporeuse pour des applications orthopédiques ou dentaires comprenant des pores fabriqués à partir d'un processus de dégagement d'hydrogène qui survient dans une solution contenant un mélange d'une poudre post-traitement aux micro-ondes et d'un liquide comprenant de la silice, dans laquelle les pores sont d'une taille dans la plage d'environ 100 µm à environ 1 mm, et dans laquelle la composition de ciment macroporeuse comprend un phosphate de magnésium qui a été irradié par des micro-ondes, la composition de ciment macroporeuse étant injectable pendant le processus de dégagement d'hydrogène.

8. Composition de ciment macroporeuse selon la revendication 7, dans laquelle le processus de dégagement d'hydrogène est une réaction entre des particules de magnésium et de l'eau présente dans un liquide.

9. Composition de ciment macroporeuse selon la revendication 7, dans laquelle la composition de ciment macroporeuse comprend un biopolymère ou un polysaccharide.

10. Composition de ciment macroporeuse selon la revendication 7, comprenant en outre des molécules de médicament dans les pores.
